# EUROPEAN PATENT APPLICATION

(11) **EP 3 338 809 A1**
(43) Date of publication of application: **27.06.2018**
(21) Application number: 16836294.5
(22) Date of filing: 28.07.2016
(51) Int. Cl.: A61L 2/22, A61L 2/24, B08B 9/032

(54) **METHOD AND DEVICE FOR DISINFECTING INNER SURFACES OF FREEZERS AND THE LIKE**

(30) Priority: 20.08.2015 BR 102015020083
(71) Applicant: Aurra Serviços Especializados Ltda., CEP 05422-030 São Paulo, SP (BR)
(72) Inventor: AGMONT E SILVA, Caio Vinicius, 04530-050 São Paulo (BR)
(74) Representative: Pons
(86) International application number: PCT/BR2016/050176
(87) International publication number: WO 2017/027942

(57) **Abstract**

A method and a device for disinfecting inner surfaces of freezers and the like includes the production of a microfilm of sanitizing solution on the inner surfaces and on the devices contained in the freezers and the like, by a method which comprises two sequential steps, a first step in which 100% of the inner surface of the internal environment of the freezer or the like is completely saturated with a mist of sanitizing solution, which is then condensed, and a second step in which the air containing this mist is passed through a filter provided with means that absorb the sanitizing solution, both steps being implemented by closed-loop recirculation means (looping).

## Description

### Field of the Invention

The present invention pertains to the disinfection and/or sanitization of cooling and freezing chambers, comprising tunnel-type freezers, spiral-type freezers and the like, encompassing the treatment of their inner surfaces as well as the equipment contained therein, such as belts, condensers and fixed and mobile parts.

### State of the Art

There is growing concern in the food industry for food safety. Frozen products have become an important component of a healthy diet. The RTE (ready-to-eat) products segment has shown a major growth potential, stimulating the growing entry of various companies onto this market.

Since the products are by large of vegetable and animal origin and milk by-products, they are highly susceptible to contamination. Accordingly, it is essential to ensure that all surfaces having contact with the product to be frozen/cooled are thoroughly sanitized and free of pathogens, mainly Listeria and Salmonella to prevent product contamination. Any shortcoming in the disinfection process along the productive chain might compromise the integrity of the products, decreasing their shelf life and exposing consumers to health problems. These may range from minor discomfort to more serious consequences, such as intoxication or death.

Sanitization theoretically reduces but does not necessarily entirely eliminate micro-organisms in inanimate objects to levels considered safe. Disinfection consists of the total elimination of fungi and bacteria in inanimate objects, but not necessarily the elimination of spores.

The chief villain of the edible products industry is Listeria monocytogenes (LM), a pathogen capable of contaminating food. LM causes a disease called listeriosis, responsible for thousands of cases of intoxication and over 260 deaths every year in the USA alone. In healthy individuals, listeriosis may cause symptoms such as flu, but in highly susceptible populations, such as the elderly and immuno-compromised individuals, it may lead to death. Various outbreaks of listeriosis are associated with the consumption of foodstuffs contaminated by LM.

LM is found in the soil, air, water, dust and is easily introduced into food-processing areas. LM can easily appear on floors, drains and on standing water in food processing units. Without due care in sanitization processes, LM can easily contaminate surfaces of equipment having contact with foodstuffs in the productive chain. LM has a unique characteristic of growing in environments with negative temperatures, which represents a major risk for the internal areas of freezers and chilled environments.

The periodicity for cleaning and disinfecting a freezer will depend on the processed product, and may be daily, weekly or half-yearly. For food with high risk of contamination such as meat byproducts, the cleaning and disinfecting process should be daily.

Freezers are sanitized by the CIP (clean-in-place) process which encompasses the steps of cleaning and disinfection/sanitization. In this process, all surfaces of the equipment are sanitized without the need for disassembly.

Cleaning is the total elimination of solid residues present on the inner surfaces of the freezer such as fat, product remains, dust and other foreign substances, generally carried out with alkaline detergents.

Disinfection or sanitization is the reduction of the number of micro-organisms on the inner surfaces of the freezers to a degree that does not compromise the safety or suitability of the product. This step may be carried out in a physical (steam-thermal) or chemical form. In the first case, the heat may be transferred to the surface using steam or hot water. The use of steam has limited application due to high costs, slowness in the process and reduction of lifespan of certain components and equipment, besides the delay in resuming work due to the time required for the freezer to return to ideal temperature.

Chemical disinfection is most used by industries. The chosen sanitizer or disinfectant must be compatible with the surface to be treated, have a broad spectrum of microbiological action, act quickly on the microorganisms, be stable and resistant to the presence of organic materials, having a low degree of corrosivity and toxicity, and low cost. Peracetic acid is the best sanitizer for current market needs.

The disinfection procedure is done by a CIP system comprising an assembly of tanks (water, cleaning product, sanitizer product), a pumping system with high flow rate and pressure, and internal dispersion apparatuses in the tank such as spray nozzles. The function of this assembly is to guarantee that the cleaning/disinfection solution reaches all points of the freezer, including the upper part and recesses thereof. The CIP system should ensure good impact of the chemical solutions with all surfaces to remove any solid residue impregnated on the walls of the freezers.

Due to the constructional features of the freezers, it is very hard for the CIP system to guarantee contact of the cleaning and disinfecting solution with all surfaces. Joining bridges between fixed and mobile parts, grooves of the conveyor belts and evaporators are areas that are hard to access. The solution is to install a very large number of sprinklers, which ultimately leads to excessive consumption of the sanitizing solution. Even with the use of the CIP, manual intervention is not discarded in the disinfection process.

The complete CIP process in a freezer or equivalent equipment consists of the following steps:
1. Defrosting;
2. Visual inspection and removal of ice by hand (defrost residues);
3. Application of foam with detergent;
4. Visual inspection to verify whether the detergent has reached all parts of the freezer;
5. Rinsing;
6. Visual inspection and use of hose to remove residual foam;
7. Rinsing the conveyor belt;
8. Application of sanitizing solution;
9. Rinsing;
10. Drying the freezer;
11. Final inspection.

In order to reduce the consumption of water and sanitizing solution, some steps of the process use recirculation of the cleaning and disinfecting solutions.

When using the physical process, where steam is utilized, the major challenge is to guarantee that the whole internal surface of the freezer attains the minimum desired temperature for the minimum necessary period.

It is important to note that the vast majority of freezers and similar equipment have no automatic CIP system and therefore the cleaning and disinfecting processes are performed manually, susceptible to human shortcomings.

Disinfection/sanitizing by chemical agent in a freezer CIP is carried out with a sanitizing solution in liquid state. This solution must come into contact with all of the inner surfaces of the freezer where the product produced comes into contact, for a minimum time, as recommended by the sanitizer manufacturer and in accordance with government regulations. Depending on the size of the freezer, the volume of sanitizing solution consumed may be dozens of m³ and the time spent may be a few hours. This means that there is enormous consumption of water, sanitizing product, power (pumps to carry out the circulation of the solution) and increased generation of effluents, which must be treated before being disposed of to the environment.

In addition to the drawbacks cited above, some disinfecting processes are still performed manually, where personnel has to enter confined spaces in order to apply the sanitizing product. This is a high-risk activity, since the environment is confined, exposing people to toxic gases from evaporation of the sanitizing products.

### Objectives of the Invention

In light of the foregoing, the first objective of the present invention is to enhance the safety of the food products by disinfecting/sanitizing 100% of the inner surfaces of freezers, cooling/freezing chambers and the like by applying a sanitizing solution that reaches all their surfaces.

Another objective is to minimize the risks to the health of the personnel, preventing the entry thereof into chambers to perform the sanitization process manually, as in most cases currently.

Another objective is to reduce the consumption of sanitizing solution, as well as minimize the problems inherent to the disposal thereof into the environment.

Another objective is to decrease the consumption of power in the disinfection processes in general.

Another objective is to reduce the time spent in the disinfection/sanitization step of the CIP process.

Another objective is to reduce the need for rinsing after disinfection.

Another objective is to disinfect the air contained inside the freezer.

Another objective is to economize water in the CIP process.

### Brief Description of the Invention

The above objectives, in addition to others, are achieved by a method comprised of two steps, the first comprising the saturation of the space inside the freezer by a mist of sanitizing solution and subsequent condensation on the inner surfaces, said mist being comprised by micro droplets having a size equal to or less than 5 microns, generated by equipment outside the freezer and introduced therein by a first looping (recirculation) system, followed by a second step in which the air saturated by biocide solution contained in said interior space is filtered by an element that absorbs the sanitizing solution and is returned to the inside of the freezer or similar by a second looping system.

According to another characteristic of the invention, said mist of sanitizing solution is produced by means of piezoelectric elements installed in a mist-producing tank and excited by a high frequency generator.

According to another characteristic of the invention, the means of dragging the mist of sanitizing solution inside the freezer is the atmospheric air.

According to another characteristic of the invention, the filtering elements, absorbing the sanitizing solution, comprise the activated carbon.

According to another characteristic of the invention, said external equipment may comprise equipment that is fixed or mounted in an external cabinet.

### Description of the Drawing

The other advantages and characteristics of the present invention will become more evident from the description of a preferred embodiment, given as an example and not as a limitation, and of the single drawing which refers thereto, which, by means of an elevational view, illustrates the apparatus of the invention associated to a cold chamber.

### Detailed Description of the Invention

As already mentioned, the process of disinfecting the surfaces of the freezer is critical to guarantee food safety. The system that is the object of the present invention will suppress the need to perform the disinfection/sanitization step by the spray process with sanitizing solution in liquid state, or by a physical disinfection process using steam and by manual disinfection process, in addition to saving time, chemical products, water, power, reduction of effluent generation and lower operating risk inherent to the current system, in which personnel have to enter the chamber to perform the process manually.

In the traditional disinfection/sanitization process, a sanitizing solution in liquid state should be sprayed under pressure inside the freezer by various diffusers conveniently positioned therein, with a view to assuring contact of the sanitizing solution on 100% of the inner surfaces of the freezer and the equipment contained therein. However, due to the constructional features of freezers, it is very hard to attain said objective.

The use of a physical process, through disinfection by high temperature with the use of steam, although guaranteeing contact with all surfaces, has the drawback of consuming much power, both to generate the steam and to cool the freezer at the end of the process, besides being time-consuming and inducing fatigue to the parts and structures of the freezer owing to the large temperature swing inherent to the process.

The invention now proposed comprises a first step in which a mist of sanitizing solution is generated, introduced inside the freezer by drag of the atmospheric air, aspirated from inside the freezer, in a first recirculation process for the total saturation of the internal environment of the freezer and consequent condensation, resulting in the formation of a microfilm of disinfectant/sanitizing solution on all existing surfaces, besides disinfecting the very air inside the freezer.

Additionally, the method of the invention comprises a second step, in which the air inside the freezer is filtered by elements that absorb the sanitizing solution, therefore reducing its concentration to a degree that enables personnel to enter inside the chamber for a final inspection thereof.

The mist of sanitizing solution has the same chemical characteristics as the liquid sanitizing solution used in the disinfection step of CIP processes. In fact, it is the same solution. The difference is that it works with a droplet size of approximately 5 microns, which increases the contact surface area. It is thus possible to occupy the entirety of the three-dimensional space, reaching 100% of the inner surfaces of the chamber with a fraction of the volume of solution that would be required if a solution in liquid state applied by spray were used.

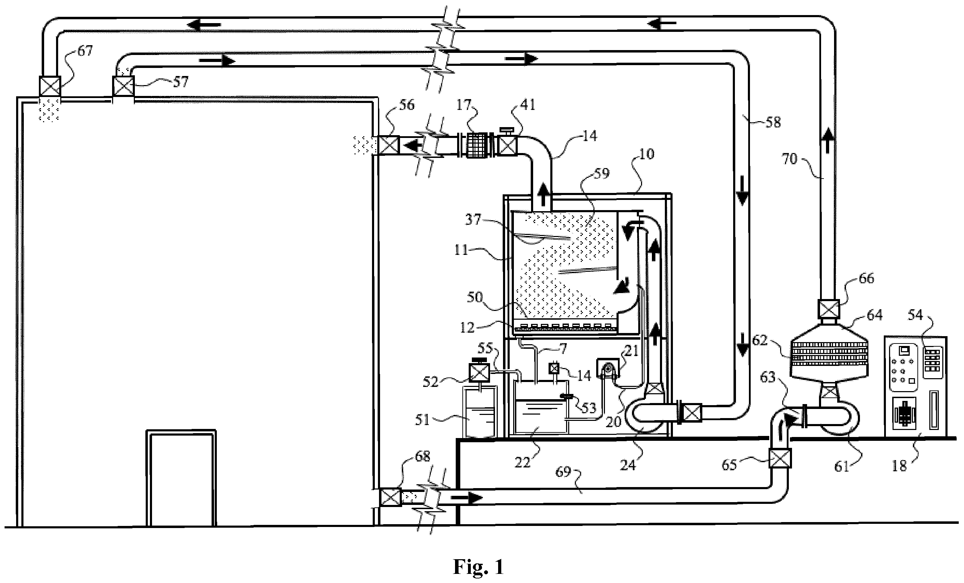

Referring now to Fig. 1, the invention comprises an apparatus outside the chamber of the freezer, comprised of mist-generating equipment, a tank for storing the sanitizing product, a tank for storing the sanitizing solution, a mist-generating tank, a first recirculation (looping) system formed by a first centrifugal fan and inlet and return ducts as well as pneumatic valves, and a second recirculation system formed by a second centrifugal fan associated to a filtering device having activated carbon elements, as well as inlet and return ducts and pneumatic valves.

The equipment generating the sanitizing mist is housed in a cabinet 10 inside of which there is a mist-generating tank 11, made of stainless steel 304, 316, 316L or any other acid corrosion resistant material.

Said tank 11 is where the liquid sanitizing solution 50 is fragmented into micro droplets. At its base there is a set of boards with piezoelectric elements 12, the amount of which depends on the desired mist output volume. Said boards must be made of stainless or corrosive solution resistant material.

Controlling the level of sanitizing solution 50 is essential for proper operation of the system, since the mist production may be adversely affected if the column of sanitizing solution above the surface of the piezoelectric elements 12 exceeds 50mm. Said control is done by an overflow system comprising a peristaltic pump 21, the sanitizing solution reservoir 22, the sanitizing solution intake pipe, the solution supply pipe, the tank for storing the sanitizing product 51, the precision dispenser 52, the level sensor of the sanitizing solution tank 53 and the water inlet solenoid valve 14.

Information from the logic controller (PLC) 54, located on the command panel 18, drives the precision dispenser pump 52 which sends the correct amount of sanitizing product contained in the sanitizing product reservoir 51 to the sanitizing product solution tank 22, through the pipe 55. Thereafter, the solenoid valve 14 opens and the solution tank 22 fills with water up to the level of the sensor 53, forming the sanitizing solution. The peristaltic pump 21 will transfer the sanitizing solution through the duct 20 to the mist-generating tank 11. Once the level of the sanitizing solution is completed, the surplus will return to the solution tank 22 through the drainage system 7. At this point, the system is ready to generate the sanitizing mist.

Once the correct level of sanitizing solution is reached in the tank 11, the PLC 54 commands the pneumatic valves 56 and 57 to open. Thereafter, the piezoelectric transducers 12 are powered by electronic circuitry that generates an ultrasonic signal to the order of hundreds of kHz to some MHz, and the size of the mist droplet produced is based on this frequency.

As shown in the drawing, the mist-generating tank 11 is further provided with anti-droplet deflectors 37 the function of which is to condense the large droplets generated in the process.

In this case, the means of dragging the mist is the very atmospheric air withdrawn from inside the freezer. The PLC 54 commands the drive of the first centrifugal fan 24, which draws the air from inside the freezer through the pipe 58 and blows it into the mist-generating box 11. This air constitutes the drag means carrying the sanitizing mist 59, transporting it through the return pipe 14 back into the internal space of the freezer through the valve 56.

This first closed-circuit recirculation process is maintained until complete saturation occurs inside the freezer, extending for an additional period according to the necessary contact time between the sanitizing solution and the inner surfaces.

Monitoring the flow rate of the mist produced is achieved by means of an in-line sensor 41 as well as visually through the glass visor 17.

The system of the invention is commanded from the control panel 18, where the electrical protection elements of the equipment (breakers, relays etc.) are located, as well as the controllers that provide for operating the equipment manually or automatically. Depending on the application, said control panel may have CLPs, timers or other controllers, according to the application.

The apparatus of the invention comprises a second closed circuit (looping) forming an air filtration system, composed by a second centrifugal fan 61, a filtering device containing activated carbon filtering elements 62, return air inlet 63, filtered air outlet 64, sanitizing sensor 65 upon return, sanitizing sensor 66 at the outlet 64, pneumatic valves 67, 68 and pipes 69, 70 forming a second closed circuit.

At the end of the disinfection/sanitization process, the mist-generating equipment is switched off, the valves 56 and 57 are closed and the process of filtering the saturated air inside the freezer begins. The purpose of the air filtering process is to reduce the concentration of sanitizing mist inside the freezer to concentrations that allow personnel to enter into the freezer for visual inspection.

In this step, the pneumatic valves 67 and 68 are opened and the second centrifugal fan 62 is driven. The air saturated with the sanitizing solution mist is drawn by the fan 61 through the aspiration pipe 69 and forced against the filtering elements 62, following through the pipe 70 and being reintroduced inside the freezer by way of the pneumatic valve 67, completing the second air recirculation circuit (looping). The concentration of sanitizer saturated in the air is measured by sensors 65 and 66, respectively before and after the air passes through the filtering elements. The sensor 65 will issue a signal to the PLC 54, when the concentration of sanitizer measured by the sensor attains the desired value, whereupon the filtration system is shut down. The PLC then commands the valves 67 and 68 to close, and also shuts down the second fan, the freezer being free for personnel to enter therein, and for operation.

Although the invention has been described based on a specific embodiment, it is understood that modifications may be introduced by persons skilled in the art, maintained within the inventive concept. Consequently, the invention is defined and delimited by the accompanying set of claims.

## Claims

1. **A METHOD FOR DISINFECTING INNER SURFACES OF FREEZERS AND THE LIKE** encompassing the treatment of 100% of their inner surfaces and of the equipment contained therein, **characterized by** comprising two sequential steps, a first step of total saturation and subsequent condensation on the inner surfaces of the internal environment of the freezer or the like by a mist of sanitizing solution by a first closed recirculation process (looping) and a second step of filtering the air saturated by sanitizing solution contained in said internal environment by the passage of this air through elements that absorb the sanitizing solution by means of a second closed recirculation process (looping).

2. The **METHOD** as claimed in claim 1, **characterized by** the fact that said first closed recirculation process comprises the removal of the air from said internal environment, its passage through a mist-generating tank and its return to said internal environment.

3. The **METHOD** as claimed in claim 1, **characterized by** the fact that the said second closed recirculation process comprises the removal of the air saturated with sanitizing mist from said internal environment, its passage through the elements that absorb the sanitizing solution and its return to said internal environment.

4. The **METHOD** as claimed in claim 1, **characterized by** the fact that the mist of sanitizing solution is produced by means of piezoelectric elements installed in a mist-generating tank and excited by a high frequency generator.

5. The **METHOD** as claimed in claims 1 or 2, **characterized by** the fact that the means of dragging said mist of sanitizing solution, from the mist-generating tank to said internal environment, consists of atmospheric air withdrawn from inside said internal environment.

6. The **METHOD** as claimed in claims 1 or 3, **characterized by** the fact that said elements that absorb the sanitizing solution comprise the activated carbon.

7. The **METHOD** as claimed in claims 1 or 4, **characterized by** the fact that the mist is comprised of droplets having a maximum size of 5 microns.

8. An **APPARATUS FOR DISINFECTING INNER SURFACES OF FREEZERS AND THE LIKE characterized by** comprising two closed-circuit recirculation systems, the first comprising an air-drawing pipe (58) from inside the freezer, or similar, a first centrifugal fan (24), a mist-generating equipment comprising a mist-generating box (11) and a return pipe (41) to the interior space of the freezer, the second system comprising a second air-drawing pipe (69) from inside the freezer or similar, a second centrifugal fan (61), a filtering device containing filtering elements (62) and a second return pipe (70) to the interior space of the freezer.

9. The **APPARATUS** as claimed in claim 8, **characterized by** the fact that said mist-generating box is provided with piezoelectric transducers (12) powered by an ultrasonic signal.

10. The **APPARATUS** as claimed in claim 8, **characterized by** comprising a control panel (18) endowed with electrical protection elements of the equipment as well as operating controllers of the equipment.
